# EUROPÄISCHE PATENTSCHRIFT

(11) **EP 1 632 270 B1**
(45) Veröffentlichungstag und Bekanntmachung des Hinweises auf die Patenterteilung: **06.02.2008**
(21) Anmeldenummer: 05014348.6
(22) Anmeldetag: 01.07.2005
(51) Int. Cl.: A61Q 5/06, A61K 8/73, A61K 8/81

(54) **Verwendung eines Haarbehandlungsmittel mit Gehalt an einer Polymerkombination**
Use of a hair treatment composition containing a polymer combination
Utilisation d'une composition de traitement capillaire comprenant une combinaison de polymères

(30) Priorität: 04.09.2004 DE 102004042848
(43) Veröffentlichungstag der Anmeldung: 08.03.2006
(73) Patentinhaber: Wella Aktiengesellschaft, 64274 Darmstadt (DE)
(72) Erfinder: Walter, Andrea, 64572 Büttelborn (DE); Runge, Martina, 68163 Mannheim (DE); Birkel, Susanne, Dr., 64285 Darmstadt (DE); Haselbauer, Iris, 64589 Stockstadt (DE)

(56) Entgegenhaltungen:
- US-A1- 2002 155 076
- US-A1- 2002 176 832
- US-A1- 2003 161 849
- US-A1- 2004 096 406

## Beschreibung

Gegenstand der Erfindung ist die Verwendung eines Haarbehandlungsmittels mit einem Gehalt an einer Kombination von mindestens drei verschiedenen Polymeren, und zwar einem anionischen Polysaccharid, einem Homo- oder Copolymer, welches aufgebaut ist aus Acryl- oder Methacrylamidoalkylsulfonsäuren oder deren Salzen und einem amphiphilen Polymer als Föhn- und/oder Finish-Gel. Bei den Mitteln handelt es sich vorzugsweise um Haarstylinggele mit einem Gehalt an Xanthan, Ammonium Acryloyldimethyltaurat/Vinylpyrrolidon Copolymer und vernetzten Copolymeren, hergestellt aus (Meth)acrylsäure und C10- bis C30-Alkylestern der (Meth)acrylsäure.

US-A-2004/0096406 offenbart kosmetische Zubereitungen, die die Polymere A, B und C des vorliegenden Haarbehandlungsmittels enthalten. Die Verwendung als Föhn und/oder Finish-Gel wird nicht erwähnt.

US-A-2002/0155076 offenbart eine Gel-Creme, die leicht auf Haut verteilbar seine Soll und die die Polymere A, B und C enthält.

Bekannte Haarstylinggele können je nach ihrer durch unterschiedliche, spezifische Inhaltsstoffe bedingten Wirksamkeit und der damit verbundenen unterschiedlichen Anwendungsweisen in zwei Gruppen unterteilt werden. Zum einen gibt es die sogenannten Finish-Gele, welche auf trockenem Haar ohne zusätzliche Wärmeeinwirkung angewendet werden, um dem Haar Definition, Struktur, Halt, Wet-Look und/oder Glanz zu verleihen. Zum anderen gibt es die sogenannten Föhn-Gele, welche auf feuchtem Haar angewendet werden und wobei die behandelten Haare anschließend unter Wärmeeinwirkung mit einem Haartrockner getrocknet werden, um eine Volumenwirkung der Frisur zu erzielen. Übliche Finish-Gele sind in der Regel nicht geeignet oder in der Lage, um auf feuchtem Haar angewendet und einen Volumeneffekt erzielen zu können. Ebenso sind übliche Föhn-Gele in der Regel nicht in der Lage, um auf trockenem Haar angewendet und die oben genannten, charakteristischen Wirkungen eines Finish-Gels zu erzielen. Es bestand daher die Aufgabe ein 2-in-1-Produkt zur Verfügung zu stellen, welches sowohl eine volumengebende Wirkung aufweist, wenn es als Föhn-Gel im feuchten Haar angewendet wird, als auch eine das Haar bzw. die Frisur strukturierende Wirkung hat, wenn es als Finish-Gel im trockenen Haar angewendet wird.

Außerdem besteht generell auf dem Gebiet der Kosmetik und insbesondere auf dem schnelllebigen und sehr modeorientierten Gebiet der Haarstylingprodukte ein Bedarf an Produkten, die sich außer durch gute Anwendungseigenschaften und Produktwirkungen auch durch weitere, den Anwender ansprechende Eigenschaften auszeichnen, z.B. besondere, neue oder ungewöhnliche Produktkonsistenzen. Die Schwierigkeit besteht darin, dass eine ungewöhnliche Konsistenz, z.B. außergewöhnliche rheologische Eigenschaften, häufig mit einer Minderung der Anwendungseigenschaften oder der primären Produktwirkungen einhergeht. Eine weitere Aufgabe bestand deshalb darin, ein Haarstylingmittel zur Verfügung zu stellen mit attraktiver, gegenüber herkömmlichen Stylingmitteln abweichender Konsistenz, ohne dass die Anwendungseigenschaften oder Produktleistungen inakzeptabel beeinträchtigt werden.

Es wurde nun gefunden, dass mit einer bestimmten Kombination von drei Polymertypen ein 2-in-1-Haarstylingmittel hergestellt werden kann und dass dieses Haarstylingmittel unter Anwendung eines bestimmten Herstellungsverfahrens eine ungewöhnliche, attraktive Konsistenz annehmen kann, ohne die Produktleistungen inakzeptabel zu beeinträchtigen.

Gegenstand der Erfindung ist die Verwendung eines Haarbehandlungsmittels gemäß Anspruch 1 mit einem Gehalt an einer Kombination von
(A) mindestens einem ersten Polymer, ausgewählt aus anionischen Polysacchariden;
(B) mindestens einem zweiten Polymer, ausgewählt aus Homo- oder Copolymeren, aufgebaut aus mindestens einer Monomerart, die ausgewählt ist aus Acryl- oder Methacrylamidoalkylsulfonsäuren oder deren Salzen und
(C) mindestens einem dritten, von (A) und (B) verschiedenen Polymer, ausgewählt aus amphiphilen Polymeren.

Das anionische Polysaccharid (A) wird vorzugsweise in einer Menge von 0,05 bis 5 Gew.% oder von 0,1 bis 1 Gew.%, insbesondere von 0,2 bis 0,5 Gew.% eingesetzt. Das zweite Polymer (B) wird vorzugsweise in einer Menge von 0,1 bis 10 Gew.% oder von 0,2 bis 5 Gew.%, insbesondere von 0,3 bis 2 Gew.% eingesetzt. Das dritte Polymer (C) wird vorzugsweise in einer Menge von 0,01 bis 5 Gew.% oder von 0,05 bis 2 Gew.%, insbesondere von 0,1 bis 0,5 Gew.% eingesetzt.

### Anionisches Polysaccharid (A)

Hydrokolloide auf Basis von Polysacchariden sind aus Saccharidmonomeren aufgebaute Polymere, wobei die Polymere z.B. durch Veretherung von Hydroxygruppen derivatisiert wein können. Anionische Polysaccharide sind aus Saccharidmonomeren aufgebaute Polymere, welche Säuregruppen aufweisen, die mit geeigneten Neutralisationsmitteln neutralisiert werden können. Nichtionische Polysaccharide weisen keine Säuregruppen auf. Das Polymer (A) kann z.B. ausgewählt sein aus Xanthan, Gellan, Carboxymethylcellulose, Hydroxypropylcellulose, Methylcellulose, Hydroxypropyl-methylcellulose, Hydroxyethylcellulose, Agar-Agar, Carrageenan, Alginate, Johannisbrotbaumkernmehl, Guar Gummi, Gummi arabicum, Karaya Gummi, Tragant, Ghatti Gummi, Pectinen und Hydroxypropylguar. Ein bevorzugtes anionisches Polysaccharid ist Xanthan (INCI-Bezeichnung: Xanthan Gum). Xanthan ist ein wasserlösliches natürliches Polysachccarid und enthält als Einzelbausteine D-Glucose, D-Mannose und D-Glucuronsäure und daneben noch Acetyl- und Pyruvylgruppen. Es handelt sich um eine β(1,4)-Glucan-Kette, bei welcher die 3-Position des Glucosemoleküls mit einer Seitenkette verbunden ist, die aus zwei Mannose-Einheiten und einer Glucuronsäure-Einheit besteht. Das Molekulargewicht M_{R} liegt vorzugsweise im Bereich von ca. 10⁵ bis 10⁸, besonders bevorzugt bis 10⁷.

### (Metha)acrylamidoalkylsulfonsäurepolymer (B)

Das erfindungsgemäß einzusetzende zweite Polymer ist ein Homo- oder Copolymer, aufgebaut aus mindestens einer Monomerart ausgewählt aus Acryl- oder Methacrylamidoalkylsulfonsäure oder deren Salzen. Geeignete Salze sind z.B. solche mit Ammonium-, Alkalimetall- oder Erdalkalimetallkationen. Das Polymer ist vorzugsweise aufgebaut aus Monomeren der allgemeinen Formel H₂C=CH-C(=O)-NH-A-SO₃H oder deren Salzen, wobei A für eine divalente C2- bis C6-, vorzugsweise für eine C3- oder C4-Kohlenwasserstoffgruppe steht, besonders bevorzugt ist die Gruppe -C(CH₃)₂-CH₂- Dieses Monomer ist vorzugsweise copolymerisiert mit mindestens einem nicht ionischen, radikalisch copolymerisierbarem Monomer, insbesondere einem Vinyllactam, besonders bevorzugt Vinylpyrrolidon. Ein besonders bevorzugtes Polymer ist eines mit der INCI-Bezeichnung Ammonium Acryloyldimethyltaurate/VP Copolymer. Ein geeignetes Handelsprodukt ist Aristoflex^{®} AVC.

### Amphiphiles Polymer (C)

Amphiphile Polymere enthalten mindestens eine hydrophile Gruppe und mindestens eine hydrophobe Gruppe wie z.B. eine Fettkette. Eine Fettkette kann z.B. eine Kohlenwasserstoffgruppe mit 8 bis 30 oder 10 bis 22 C-Atomen sein. Als amphiphile Polymere können Assoziativverdicker eingesetzt werden. In dem amphiphilen Polymer können die hydrophoben Molekülbereiche gegenüber dem Rest der Polymerkette zahlenmäßig reduziert sein. Sie können sich seitlich an der Kette befinden, statistisch verteilt sein (statistische Copolymere), in Form von Blöcken verteilt sein (Blockcopolymere oder sequentielle Polymere) oder als Pfropfzweige vorliegen (Pfropfcopolymere). Die amphiphilen Polymere können wasserlöslich, wasserdispergierbar oder in Wasser quellbar sein. Es kann sich um Polymere natürlicher Herkunft, die gegebenenfalls modifiziert sind, oder um synthtische Polymere handeln. Synthetische Polymere können z.B. durch radikalische Polymerisation (z.B. Vinyl- oder Acrylatpolymere) oder durch Polykondensation (z.B. Polyester oder Polyurethane) hergestellt sein. Es können auch Gemische der genannten Polymere eingesetzt werden. Die amphiphilen Polymere können ionisch oder nichtionisch sein, wobei anionische und nichtionische bevorzugt sind.

Die amphiphilen Polymere (C) sind vorzugsweise ausgewählt aus
(1) Copolymeren von Acryl- oder Methacrylsäure und Monomeren, die mindestens eine Fettkette aufweisen;
   Copolymere dieses Typs sind z.B.
   - vernetzte Acrylsäure/C10-30-Alkylacrylat Copolymere, z.B. Pemulen^{®} TR1, Pemulen^{®} TR2, Carbopol^{®} 1382, Carbopol^{®} 1342, Carbopol^{®} ETD 2020;
   - (Meth)acrylsäure/Ethylacrylat/Alkylacrylat Copolymere, z.B. Acusol^{®} 823, Imperon R^{®};
   - vernetzte Acrylsäure/Vinylisododecanoat Copolymere, z.B. Stabylen^{®} 30;
   - Acrylsäure/Vinylpyrrolidon/Laurylmethacrylat Terpolymere, z.B. Acrylidone LM^{®}, ACP-1184^{®}, ACP-1194^{®};
   - Acrylsäure/Lauryl(meth)acrylat Copolymere, z.B. Coatex SX^{®};
   - (Meth)acrylsäure/Alkylacrylat/Alkyl(polyethoxy)allylether, z.B. Rheovis^{®} CR, CR3, CR2 und CRX;
   - Methacrylsäure/Ethylacrylat/Stearyl(polyethoxy)allylether, z.B. Salcare^{®} SC90 und SC80;
   - (Meth)acrylsäure/Ethylacrylat/polyethoxyliertes Laurylacrylat Terpolymer, z.B. Rheo 2000^{®};
   - Methacrylsäure/Ethylacrylat/polyethoxyliertes Stearylmethacrylat Terpolymer, z.B. Acrysol^{®} 22, Acrysol^{®} 25 und DW-1206A von Rhom&Haas;
   - Methacrylsäure/polyethoxyliertes Nonylphenolacrylat Copolymer, z.B. Rheo 3000^{®};
   - Acrylsäure/polyethoxyliertes Stearyl- oder Cetylmonoitaconat, z.B. Structure^{®} 2001 und 3001
   - Copolymere von Methacrylsäure, Butylacrylat und einem hydrophoben Monomer mit mindestens einer Fettkette, z.B. das Produkt 8069-146A von National Starch;
   - Terpolymere von Acrylsäure/C15-Alkylacrylat/Polyethylenglykolacrylat (28 mol Ethylenoxid), z.B. Dapral GE 202^{®}
   - Partielle Fettsäureestersalze eines Copolymers von Acrylsäure/Dimethylethanolamin, z.B. Dapral GE 202 DMA^{®}
   - Copolymere von Acrylsäure, Acrylat und einem amphiphilen Monomer, das eine Fettkette mit Urethangruppe aufweist, z.B. Additol VXW 1312^{®}
   - Mit hydrophoben Gruppen mit Fettkette modifizierte Acrylcopolymere wie z.B. Acusol 102^{®};
(2) Polysacchariden, die mit Gruppen modifiziert sind, die mindestens eine Fettkette aufweisen;
   Copolymere dieses Typs sind z.B.
   - Celluloseverbindungen oder deren Derivate, die mit Gruppen modifiziert sind, die mindestens eine Fettkette aufweisen, z.B. Alkyl-, Arylalkyl- oder Alkylarylgruppen oder deren Gemische, wobei die Alkylgruppen 8 bis 22 C-Atome aufweisen;
   - Nichtionische Alkylhydroxyethylcellulosen, z.B. Natrosol^{®} Plus Grade 330 CS, Polysurf^{®} 67 und ADX 401 (C16-Alkyl) von Aqualon;
   - Quaternisierte Alkylhydroxyalkylcellulosen, z.B. Quatrisoft^{®} LM 200, Quatrisoft^{®} LM-X 529-18-A Quatrisoft^{®} LM-X 529-18-B (C12-Alkyl), Quatrisoft^{®} LM-X 529-8 (C18-Alkyl), Crodacel^{®} QM, Crodacel^{®} QL (C12-Alkyl), Cordacel^{®} QS (C18-Alkyl);
   - Nichtionische Nonoxynylhydroxyethylcellulosen, z.B. Amercell^{®} HM-1500;
   - Nichtionische Alkylcellulosen, z.B. Bermocell^{®} EHM 100;
   - Poly-c₁₂₋₁₈-alkoholsaccharide, z.B. Emulsan^{®} (D-Galactosamin/Aminouronsäure-Gemisch), Biosan^{®} LPS-50;
   - Mit einer Fettkette modifiziertes Hydroxyalkylguargum, z.B. Esaflor^{®} HM 22 (mit C22-Alkyl modifiziert), Miracare^{®} XC 95-3 ((mit C14-Alkyl modifiziert), RE 205-146 (mit C20-Alkyl modifiziert) von Rhone-Poulenc,
(3) Copolymeren von Maleinsäureanhydrid oder einem ihrer Derivate und Monomeren, die mindestens eine Fettkette aufweisen;
   Copolymere dieses Typs sind z.B.
   - N-Octadecylvinylether/Maleinsäureanhydrid Copolymere, z.B. Gantrez^{®} AN-8194
   - Vinylacetat/Isobutylmonomaleat/Vinylneodecanoat Terpolymer, z.B. die Produkte ACV-4033 und 9649-147 von ISP, Meypro-Fix 509^{®}, Densodrin^{®} BA, Lipoderm Liquor FP;
(4) Polyurethanen und deren Derivaten, die Gruppen tragen, die mindestens eine Fettkette aufweisen; z.B. Rheolate^{®} 204, 205, 208, 210, 255 und 278; Bermodol Pur^{®} 2130; Acrysol^{®} SCT-275, Acrysol^{®} RM-870, Acrysol^{®} RM-825, Acrysol^{®} 44, Acrysol 46^{®}, DW-1206 B, DW-1206 F, SW-1206 G und DW-1206 J von Rohm & Haas; Dapral^{®} T 212, SER-AD FX 1100, Borchigel^{®} L.75.N;
(5) Copolymeren von Crotonsäure und Monomeren, die mindestens eine Fettkette aufweisen; z.B. Vinylacetat/Crotonsäure/ Allylstearat Terpolymere;
(6) Copolymeren von N-Vinylpyrrolidon und Monomeren, die mindestens eine Fettkette aufweisen; z.B. den mit einer Alkylgruppe substituierten Olefinen, die eine langkettige Kohlenwasserstoffgruppe enthalten, z.B. Antaron^{®} V216 und Antaron^{®} V220 von ISP;
(7) nichtionischen Copolymeren von Acryl- oder Methacrylsäurealkylestern mit C₁₋₆-Alkylgruppen und amphiphilen Monomeren mit Fettkette; z.B. Copolymeren von Methylmethacrylat und polyethoxyliertem Stearylacrylat, z.B. Antil^{®} 208;
(8) nichtionischen Copolymeren von hydrophilen Acrylaten oder Methacrylaten und hydrophoben Monomeren mit Fettkette; z.B. Copolymere von Polyethylenglykolmethacrylat und Alkylmethacrylat.

Das erfindungsgemäße Mittel wird bevorzugt in einem wässrigen, einem alkoholischen oder in einem wässrig-alkoholischen Medium mit vorzugsweise mindestens 10 Gew.%, besonders bevorzugt mindestens 50 Gew.% Wasser und vorzugsweise maximal 40 Gew.% Alkohol konfektioniert. Als Alkohole können insbesondere die für kosmetische Zwecke üblicherweise verwendeten niederen Monoalkohole mit 1 bis 4 C-Atomen wie z.B. Ethanol und Isopropanol enthalten sein.

Besonders bevorzugte amphiphile Polymere (C) sind die Copolymere von Acryl- oder Methacrylsäure und Alkylestern der Acryl- oder Methacrylsäure, insbesondere vernetzte Acrylsäure/C10-30-Alkylacrylat Copolymere, z.B. Pemulen^{®} TR1, Pemulen^{®} TR2, Carbopol^{®} 1382, Carbopol^{®} 1342, Carbopol^{®} ETD 2020.

Eine besondere Ausführungsform betrifft ein Haarbehandlungsmittel in Gelform mit einem Gehalt an einer Kombination von
(A) 0,1 bis 1 Gew.% Xanthan,
(B) 0,2 bis 5 Gew.% Ammonium Acryloyldimethyltaurat/Vinylpyrrolidon Copolymer und
(C) 0,05 bis 2 Gew.% an vernetzten Copolymeren, hergestellt aus mindestens einer ersten Monomerart, welche ausgewählt ist aus Acrylsäure und Methacrylsäure und mindestens einer zweiten Monomerart, welche ausgewählt ist aus C10- bis C30-Alkylestern der Acryl- oder Methacrylsäure.

In einer bevorzugten Ausführungsform enthält das Gel zur weiteren Glanzverbesserung mehrwertige Alkohole (Polyole), vorzugsweise solche mit 2 bis 6 C-Atomen und mit 2 bis 6 Hydroxygruppen in einer Menge von 0,1 bis 15, bevorzugt von 1 bis 10 Gew.%. Besonders bevorzugt sind Glycerin, Ethylenglykol, Propylenglykol, insbesondere 1,2-Propylenglykol und Sorbitol.

Die Säuregruppen enthaltende Polymere sind vorzugsweise durch organische oder anorganische Basen ganz oder teilweise, vorzugsweise zu 50 bis 100% neutralisiert. Geeignete Neutralisationsmittel sind primäre oder sekundäre Amine, insbesondere Aminoalkanole mit vorzugsweise 1 bis 10 C-Atomen und 1 bis 3 Hydroxygruppen wie z.B. Aminomethylpropanol (AMP), Triethanolamin, Tetrahydroxypropylethylendiamin oder Monoethanolamin, aber auch anorganische Basen wie Ammoniak, NaOH, KOH u.a..

Das erfindungsgemäße Mittel weist vorzugsweise einen pH-Wert im Bereich von 6 bis 9 auf.

Das erfindungsgemäße Haarbehandlungsmittel liegt vorzugsweise in Form eines Gels vor. Die Viskosität des Gels beträgt vorzugsweise von 1000 bis 100.000, besonders bevorzugt von 10.000 bis 50.000 mPa s, ganz besonders bevorzugt von 25.000 bis 35.000 mPa s , gemessen als dynamische Viskositätsmessung mit einem HAAKE VT-550 Rheometer, Messkörper SV-DIN bei einer Temperatur von 25°C und einer Schergeschwindigkeit von 50 S⁻¹.

Das Gel kann farblos oder eingefärbt sein. Es kann klar, transparenten oder zumindest durchscheinend sein, es kann aber auch undurchsichtig und trüb sein, z.B. aufgrund eines Gehaltes an Pigmenten, insbesondere Perlglanzpigmenten oder sonstigen unlöslichen Stoffen.

In einer weiteren Ausführungsform enthält das erfindungsgemäß Mittel zusätzlich mindestens ein filmbildendes und/ oder haarfestigendes Polymer. Das filmbildende oder haarfestigende Polymer kann nichtionisch, anionisch, kationisch, zwitterionisch oder amphoter sein, ist aber vorzugsweise nichtionisch oder anionisch. Es kann ein synthetisches oder ein natürliches Polymer sein. Unter natürlichen Polymeren werden auch chemisch modifizierte Polymere natürlichen Ursprungs verstanden. Bevorzugt sind insbesondere solche Polymere, die eine ausreichende Löslichkeit in Wasser, Alkohol oder Wasser/Alkohol-Gemischen besitzen, um in dem erfindungsgemäßen Mittel in vollständig gelöster Form vorzuliegen. Unter haarfestigenden Polymeren werden erfindungsgemäß solche Polymere verstanden, die bei Anwendung in 0,01 bis 5%iger wässriger, alkoholischer oder wässrig-alkoholischer Lösung oder Dispersion in der Lage sind, auf dem Haar eine haarfestigende Wirkung zu erzielen. Die filmbildenden oder haarfestigenden Polymere werden in einer Menge von vorzugsweise 0,01 bis 30 oder 0,1 bis 15, besonders bevorzugt von 0,5 bis 10 Gew.% eingesetzt.

Geeignete synthetische, nichtionische haarfestigende Polymere sind Homo- oder Copolymere, die aus mindestens einem der folgenden Monomere aufgebaut sind: Vinylpyrrolidon, Vinylcaprolactam, Vinylester wie z.B. Vinylacetat, Vinylalkohol, Acrylamid, Methacrylamid, Alkyl- und Dialkylacrylamid, Alkyl- und Dialkylmethacrylamid, Dialkylaminoalkylmethacrylamid, Dialkylaminoalkylacrylamid, Alkylacrylat, Alkylmethacrylat, Propylenglykol oder Ethylenglykol, wobei die Alkylgruppen dieser Monomere vorzugsweise C1- bis C7-Alkylgruppen, besonders bevorzugt C1- bis C3-Alkylgruppen sind. Geeignet sind z.B. Homopolymere des Vinylcaprolactams, des Vinylpyrrolidons oder des N-Vinylformamids. Weitere geeignete haarfestigende Polymere sind z.B. Copolymerisate aus Vinylpyrrolidon und Vinylacetat, Terpolymere aus Vinylpyrrolidon, Vinylacetat und Vinylpropionat, Terpolymere aus Vinylpyrrolidon, Vinylcaprolactam und Dialkylaminoalkyl(meth)acrylat, Terpolymere aus Vinylpyrrolidon, Vinylcaprolactam und Dialkylaminoalkyl(meth)-acrylamid, Polyacrylamide, Polyvinylalkohole, sowie haarfestigende Polyethylenglykol/Polypropylenglykol Copolymere. Besonders bevorzugte nichtionische Polymere sind Polyvinylpyrrolidon und Polyvinylpyrrolidon/Vinylacetat Copolymere. Bevorzugt sind nichtionische Vinyllactam Homo- oder Copolymere. Geeignete Vinyllactame sind z.B. Vinylcaprolactam und Vinylpyrrolidon. Besonders bevorzugt sind Polyvinylpyrrolidon, Polyvinylcaprolactam und Vinylpyrrolidon/Vinylacetat Copolymere. Bevorzugte Handelsprodukte sind Luviskol^{®} VA 37 und Luviskol^{®} VA 64.

Geeignete anionische haarfestigende Polymere können natürliche oder synthetische Homo- oder Copolymere mit Säuregruppen enthaltenden Monomereinheiten sein, welche gegebenenfalls mit Comonomeren, die keine Säuregruppen enthalten, copolymerisiert sind. Die Säuregruppen sind vorzugsweise ausgewählt aus -COOH, -SO₃H, -OSO₃H, -OPO₂H und -OPO₃H₂, von denen die Carbonsäuregruppen bevorzugt sind. Die Säuregruppen können unneutralisiert, teilweise oder vollständig neutralisiert vorliegen. Sie liegen vorzugsweise zu 50 bis 100% in anionischer bzw. neutralisierter Form vor. Als Neutralisationsmittel können die oben genannten Neutralisationsmittel verwendet werden. Geeignete Monomere sind ungesättigte, radikalisch polymerisierbare Verbindungen, welche mindestens eine Säuregruppe tragen, insbesondere Carboxyvinylmonomere. Geeignete Säuregruppen enthaltende Monomere sind beispielsweise Acrylsäure, Methacrylsäure, Crotonsäure, Maleinsäure bzw. Maleinsäureanhydrid oder deren Monoester, Aldehydocarbonsäuren oder Ketocarbonsäuren.

Nicht mit Säuregruppen substituierte Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylester, Vinylalkohol, Propylenglykol oder Ethylenglykol, aminsubstituierte Vinylmonomere wie z.B. Dialkylaminoalkylacrylat, Dialkylaminoalkylmethacrylat, Monoalkylaminoalkylacrylat und Monoalkylaminoalkylmethacrylat, wobei die Alkylgruppen dieser Monomere vorzugsweise C1- bis C7-Alkylgruppen, besonders bevorzugt C1- bis C3-Alkylgruppen sind.

Geeignete anionische Polymere sind insbesondere (von der Komponente (A) verschiedene) Copolymere der Acrylsäure oder Methacrylsäure mit Monomeren ausgewählt aus Acrylsäure- oder Methacrylsäureestern, Acrylamiden, Methacrylamiden und Vinylpyrrolidon, Homopolymere der Crotonsäure sowie Copolymere der Crotonsäure mit Monomeren ausgewählt aus Vinylestern, Acrylsäure- oder Methacrylsäureestern, Acrylamiden und Methacrylamiden. Ein geeignetes natürliches Polymer ist beispielsweise Schellack.

Bevorzugte anionische Polymere sind vernetzte oder unvernetzte Vinylacetat/Crotonsäure Copolymere. Ebenso bevorzugt sind partialveresterte Copolymere zwischen Vinylmethylether und Maleinsäureanhydrid. Weitere geeignete anionische Polymere sind z.B. Terpolymere aus Acrylsäure, Alkylacrylat und N-Alkylacrylamid, insbesondere Acrylsäure/Ethylacrylat/N-t-Butylacrylamid Terpolymere oder Terpolymere aus Vinylacetat, Crotonat und Vinylalkanoat, insbesondere Vinylacetat/Crotonat/Vinylneodecanoat Copolymere.

Geeignete filmbildende amphotere Polymere, sind Polymere, welche neben sauren oder anionischen Gruppen als weitere funktionelle Gruppen basische oder kationische Gruppen, insbesondere primäre, sekundäre, tertiäre oder quaternäre Amingruppen enthalten. Beispiele hierfür sind Copolymere gebildet aus Alkylacrylamid (insbesondere Octylacrylamid), Alkylaminoalkylmethacrylat (insbesondere t-Butylaminoethylmethacrylat) und zwei oder mehr Monomeren ausgewählt aus Acrylsäure, Methacrylsäure oder deren Ester, wobei die Alkylgruppen 1 bis 4 C-Atome enthalten, mindestens eines der Monomere eine Säuregruppe aufweist und wie sie z.B. unter dem Handelsnamen Amphomer^{®} oder Amphomer^{®} LV-71 der Firma NATIONAL STARCH, USA erhältlich sind.

Weitere Beispiele für geeignete haarfestigende Polymere sind Copolymere von Acrylsäure, Methylacrylat und Methacrylamidopropyltrimethylammoniumchlorid (INCI-Bezeichnung: Polyquaternium-47), Copolymere aus Acrylamidopropyltrimethylammoniumchlorid und Acrylaten, Copolymere aus Acrylamid, Acrylamidopropyltrimethylammoniumchlorid, 2-Amidopropylacrylamidsulfonat und Dimethylaminopropylamin (INCI-Bezeichnung: Polyquaternium-43) oder Chitosane. Geeignet sind auch Polymere mit Betaingruppen tragenden Monomeren wie z.B. Copolymere aus Methacryloylethylbetain und zwei oder mehr Monomeren von Acrylsäure oder deren einfachen Estern, bekannt unter der INCI-Bezeichnung Methacryloyl Ethyl Betaine/Acrylates Copolymer.

Kationische Polymere sind Polymere mit kationischen oder kationisierbaren Gruppen, insbesondere primären, sekundären, tertiären oder quaternären Amingruppen. Die kationische Ladungsdichte beträgt vorzugsweise 1 bis 7 meq/g. Bei den geeigneten kationischen Polymeren handelt es sich vorzugsweise um haarfestigende oder um haarkonditionierende Polymere. Geeignete Polymere enthalten vorzugsweise quaternäre Amingruppen. Die kationischen Polymere können Homo- oder Copolymere sein, wobei die quaternären Stickstoffgruppen entweder in der Polymerkette oder vorzugsweise als Substituent an einem oder mehreren der Monomeren enthalten sind. Die Ammoniumgruppen enthaltenden Monomere können mit nicht kationischen Monomeren copolymerisiert sein. Geeignete kationische Monomere sind ungesättigte, radikalisch polymerisierbare Verbindungen, welche mindestens eine kationische Gruppe tragen, insbesondere ammoniumsubstituierte Vinylmonomere wie z.B. Trialkylmethacryloxyalkylammonium, Trialkylacryloxyalkylammonium, Dialkyldiallylammonium und quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, Alkylvinylpyridinium, oder Alkylvinylpyrrolidon Salze. Die Alkylgruppen dieser Monomere sind vorzugsweise niedere Alkylgruppen wie z.B. C1- bis C7-Alkylgruppen, besonders bevorzugt C1- bis C3-Alkylgruppen.

Die Ammoniumgruppen enthaltenden Monomere können mit nicht kationischen Monomeren copolymerisiert sein. Geeignete Comonomere sind z.B. Acrylamid, Methacrylamid, Alkyl- und Dialkylacrylamid, Alkyl- und Dialkylmethacrylamid, Alkylacrylat, Alkylmethacrylat, Vinylcaprolacton, Vinylcaprolactam, Vinylpyrrolidon, Vinylester, z.B. Vinylacetat, Vinylalkohol, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Geeignete Polymere mit quaternären Amingruppen sind z.B. die im CTFA Cosmetic Ingredient Dictionary unter den Bezeichnungen Polyquaternium beschriebenen Polymere wie Methylvinylimidazoliumchlorid/Vinylpyrrolidon Copolymer (Polyquaternium-16) oder quaternisiertes Vinylpyrrolidon/Dimethylaminoethylmethacrylat Copolymer (Polyquaternium-11) sowie quaternäre Silikonpolymere bzw. -oligomere wie z.B. Silikonpolymere mit quaternären Endgruppen (Quaternium-80).

Bevorzugte kationische Polymere auf synthetischer Basis: Poly(dimethyldiallylammoniumchlorid); Copolymere aus Acrylamid und Dimethyldiallylammoniumchlorid; quaternäre Ammoniumpolymere, gebildet durch die Reaktion von Diethylsulfat und einem Copolymer aus Vinylpyrrolidon und Dimethylaminoethylmethacrylat, insbesondere Vinylpyrrolidon/Dimethylaminoethylmethacrylat Methosulfat Copolymer (z.B. Gafquat^{®} 755 N, Gafquat^{®} 734); quaternäre Ammoniumpolymere aus Methylvinylimidazoliumchlorid und Vinylpyrrolidon (z.B. LUVIQUAT^{®} HM 550); Polyquaternium-35; Polyquaternium-57; Polymer aus Trimethylammonium-ethyl-methacrylatchlorid; Terpolymere aus Dimethyldiallylammoniumchlorid, Natriumacrylat und Acrylamid (z.B. Merquat^{®} Plus 3300); Copolymere aus Vinylpyrrolidon, Dimethylaminopropylmethacrylamid und Methacryloylaminopropyllauryldimethylammoniumchlorid; Terpolymere aus Vinylpyrrolidon, Dimethylaminoethylmethacrylat und Vinylcaprolactam (z.B. Gaffix^{®} VC 713); Vinylpyrrolidon/Methacrylamidopropyltrimethylammoniumchlorid Copolymere (z.B. Gafquat^{®} HS 100); Copolymere aus Vinylpyrrolidon und Dimethylaminoethylmethacrylat; Copolymere aus Vinylpyrrolidon, Vinylcaprolactam und Dimethylaminopropylacrylamid; Poly- oder Oligoester, aufgebaut aus mindestens einer ersten Monomerart, die ausgewählt ist aus mit mindestens einer quaternären Ammoniumgruppe substituierten Hydroxysäure; endständig mit quaternären Ammoniumgruppen substituierte Dimethylpolysiloxane.

Geeignete kationische Polymere, die von natürlichen Polymeren abgeleitet sind, sind insbesondere kationische Derivate von Polysacchariden, beispielsweise kationische Derivate von Cellulose, Stärke oder Guar. Geeignet sind weiterhin Chitosan und Chitosanderivate. Kationische Polysaccharide haben z.B. die allgemeine Formel

G-O-B-N⁺R^{a}R^{b}R^{c} X⁻

G ist ein Anhydroglucoserest, beispielsweise Stärke- oder Celluloseanhydroglucose;
B ist eine divalente Verbindungsgruppe, beispielsweise Alkylen, Oxyalkylen, Polyoxyalkylen oder Hydroxyalkylen;
R^{a}, R^{b} und R^{c} sind unabhängig voneinander Alkyl, Aryl, Alkylaryl, Arylalkyl, Alkoxyalkyl oder Alkoxyaryl mit jeweils bis zu 18 C-Atomen, wobei die Gesamtzahl der C-Atome in R^{a}, R^{b} und R^{c} vorzugsweise maximal 20 ist;
X ist ein übliches Gegenanion, beispielsweise ein Halogen, Acetat, Phosphat, Nitrat oder Alkylsulfat, vorzugsweise ein Chlorid. Kationische Cellulosen sind z.B. solche mit den INCI-Bezeichnungen Polyquaternium-10 oder Polyquaternium-24. Ein geeignetes kationisches Guarderivat hat z.B. die INCI-Bezeichnung Guar Hydroxypropyltrimonium Chloride.

Besonders bevorzugte kationaktive Stoffe sind Chitosan, Chitosansalze und Chitosanderivate. Bei den erfindungsgemäß einzusetzenden Chitosanen handelt es sich um vollständig oder partiell deacetylierte Chitine. Das Molekulargewicht kann über ein breites Spektrum verteilt sein, beispielsweise von 20.000 bis ca. 5 Millionen g/mol, z.B. von 30.000 bis 70.000 g/mol. Vorzugsweise liegt das Molekulargewicht jedoch über 100.000 g/mol, besonders bevorzugt von 200.000 bis 700.000 g/mol. Der Deacetylierungsgrad beträgt vorzugsweise 10 bis 99%, besonders bevorzugt 60 bis 99%. Ein bevorzugtes Chitosansalz ist Chitosoniumpyrrolidoncarboxylat, z.B. Kytamer^{®} PC mit einem Molekulargewicht von ca. 200.000 bis 300.000 g/mol und Deacetylierung von 70 bis 85%. Als Chitosanderivate kommen quaternierte, alkylierte oder hydroxyalkylierte Derivate, z.B. Hydroxyethyl-, Hydroxypropyl- oder Hydroxybutylchitosan in Betracht. Die Chitosane oder Chitosanderivate liegen vorzugsweise in neutralisierter oder partiell neutralisierter Form vor. Der Neutralisationsgrad liegt vorzugsweise bei mindestens 50%, besonders bevorzugt zwischen 70 und 100%, bezogen auf die Anzahl der freien Basengruppen. Als Neutralisationsmittel können prinzipiell alle kosmetisch verträglichen anorganischen oder organischen Säuren verwendet werden wie beispielsweise Ameisensäure, Weinsäure, Äpfelsäure, Milchsäure, Zitronensäure, Pyrrolidoncarbonsäure, Salzsäure u.a., von denen die Pyrrolidoncarbonsäure besonders bevorzugt ist.

Bevorzugte kationische Polymere auf natürlicher Basis: kationische Cellulosederivate aus Hydroxyethylcellulose und Diallyldimethylammoniumchlorid; kationische Cellulosederivate aus Hydroxyethylcellulose und mit Trimethylammonium substituiertem Epoxid; Chitosan und dessen Salze; Hydroxyalkylchitosane und deren Salze; Alkyl-hydroxyalkylchitosane und deren Salze; N-Hydroxyalkylchitosanalkylether.

In einer besonderen Ausführungsform ist das erfindungsgemäße Mittel zur gleichzeitigen Festigung und temporären Haarfärbung geeignet und enthält zusätzlich mindestens ein temporär haarfärbendes Pigment. Unter temporärer Haarfärbung wird eine Farbänderung von menschlichen Haaren verstanden, die bis zur nächsten Haarwäsche hält und durch Waschen der Haare mit üblichen Shampoos wieder entfernt werden kann. Die Pigmente sind vorzugsweise in einer Menge von 0,01 bis 25 Gew.%, besonders bevorzugt in einer Menge von 5 bis 15 Gew.% enthalten. Bei den Pigmenten handelt es sich vorzugsweise nicht um Nano- sonder um Mikropigmente. Die bevorzugte Teilchengröße beträgt 1 bis 200 µm, insbesondere 3 bis 150 µm, besonders bevorzugt 10 bis 100 µm.

Die Pigmente sind im Anwendungsmedium praktisch unlösliche Farbmittel und können anorganisch oder organisch sein. Auch anorganisch-organische Mischpigmente sind möglich. Bevorzugt sind anorganische Pigmente. Der Vorteil der anorganischen Pigmente ist deren ausgezeichnete Licht-, Wetter- und Temperaturbeständigkeit. Die anorganischen Pigmente können natürlichen Ursprungs sein, beispielsweise hergestellt aus Kreide, Ocker, Umbra, Grünerde, gebranntem Terra di Siena oder Graphit. Bei den Pigmenten kann es sich um Weißpigmente wie z.B. Titandioxid oder Zinkoxid, um Schwarzpigmente wie z.B. Eisenoxidschwarz, Buntpigmente wie z.B. Ultramarin oder Eisenoxidrot, um Glanzpigmente, Metalleffekt-Pigmente, Perlglanzpigmente sowie um Fluoreszenz- oder Phosphoreszenzpigmente handeln, wobei vorzugsweise mindestens ein Pigment ein farbiges, nicht-weißes Pigment ist. Geeignet sind Metalloxide, -hydroxide und -oxidhydrate, Mischphasenpigmente, schwefelhaltige Silicate, Metallsulfide, komplexe Metallcyanide, Metallsulfate, -chromate und -molybdate sowie die Metalle selbst (Bronzepigmente). Geeignet sind insbesondere Titandioxid (CI 77891), schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510), Carmine (Cochineal). Besonders bevorzugt sind Pigmente auf Mica- bzw. Glimmerbasis welche mit einem Metalloxid oder einem Metalloxychlorid wie Titandioxid oder Wismutoxychlorid sowie gegebenenfalls weiteren farbgebenden Stoffen wie Eisenoxiden, Eisenblau, Ultramarine, Carmine etc. beschichtet sind und wobei die Farbe durch Variation der Schichtdicke bestimmt sein kann. Derartige Pigmente werden beispielsweise unter den Handelsbezeichnung Rona^{®}, Colorona^{®}, Dichrona^{®} und Timiron^{®} von der Firma Merck, Deutschland vertrieben. Organische Pigmente sind beispielsweise die natürlichen Pigmente Sepia, Gummigutt, Knochenkohle, Kasseler Braun, Indigo, Chlorophyll und andere Pflanzenpigmente. Synthetische organische Pigmente sind beispielsweise Azo-Pigmente, Antrachinoide, Indigoide, Dioxazin-, Chinacridon-, Phtalocyanin-, Isoindolinon-, Perylen- und Perinon-, Metallkomplex-, Alkaliblau- und Diketopyrrolopyrrol-Pigmente.

In einer weiteren Ausführungsform enthalten die erfindungsgemäßen Mittel zusätzlich mindestens eine Fettkomponente. Die Fettkomponenten können enthalten sein in einer Menge von z.B. 0,05 bis 15 Gew.%. Fettkomponenten sind hydrophobe, im wesentlichen wasserunlösliche Stoffe und können z.B. ausgewählt sein aus
- Silikonverbindungen, welche ausgewählt sind aus Silikonwachsen, Silikonölen, cyclischen Dimethylsiloxanen, linearen Polydimethylsiloxanen, Blockpolymeren aus Polydimethylsiloxan und Polyethylenoxid und/oder Polypropylenoxid, Polydimethylsiloxanen mit end- oder seitenständigen Polyethylenoxid- oder Polypropylenoxidresten, Polydimethylsiloxanen mit endständigen Hydroxylgruppen, phenylsubstituierten Polydimethylsiloxanen, Silikonemulsionen, Silkonelastomeren, Silikongums und aminosubstituierten Silikonen und
- Ölen und Wachsen, welche ausgewählt sind aus Paraffinwachsen, Polyolefinwachsen, Wollwachs, Wollwachsalkoholen, Candelillawachs, Olivenwachs, Carnaubawachs, Japanwachs, Apfelwachs, gehärteten Fetten, Fettsäureestern, Fettsäureglyceriden, Fettsäuretriglyceriden, Polyethylenglykolwachsen, Mineralölen, Isoparaffinölen, Paraffinölen, Squalan, Sonnenblumenöl, Kokosöl, Rizinusöl, Lanolinöl, Jojobaöl, Maisöl und Sojaöl.

Das erfindungsgemäße Mittel kann darüber hinaus die für Haarbehandlungsmittel üblichen Zusatzbestandteile enthalten, z.B. Netzmittel oder Emulgatoren aus den Klassen der nichtionischen, anionischen, kationischen oder amphoteren oberflächenaktiven Tenside, wie ethoxylierte Fettalkohole, Fettalkoholsulfate, Alkylbenzolsulfonate, Alkyltrimethylammoniumsalze, Alkylbetaine, in einer Menge von 0,1 bis 15 Gew.%; Feuchthaltemittel; Parfümöle in einer Menge von 0,01 bis 1 Gew.%; Trübungsmittel, wie z.B. Ethylenglykoldistearat, in einer Menge von etwa 0,2 bis 5,0 Gew.%; Perlglanzmittel, wie z.B. ein Gemisch aus Fettsäuremonoalkylolamid und Ethylenglykoldistearat, in einer Menge von etwa 1,0 bis 10 Gew.%; bakterizide und fungizide Wirkstoffe wie z.B. 2,4,4-Trichlor-2-hydroxydiphenylether oder Methylchlorisothiazolinon, in einer Menge von 0,01 bis 1,0 Gew.%; Puffersubstanzen, wie z.B. Natriumcitrat oder Natriumphosphat, in einer Menge von 0,1 bis 1,0 Gew.%; Anfärbestoffe, wie z.B. Fluorescein Natriumsalz, in einer Menge von etwa 0,1 bis 1,0 Gew.%; Pflegestoffe, wie z.B. Betain, Panthenol, Pflanzen- und Kräuterextrakte, Protein- und Seidenhydrolysate, Lanolinderivate, in einer Menge von 0,1 bis 5 Gew.%; Lichtschutzmittel, Antioxidantien, Radikalfänger, Antischuppenwirkstoffe, in einer Menge von etwa 0,01 bis 4 Gew.%; Fettalkohole, Konsistenzgeber, Glanzgeber, Vitamine, Weichmacher, Kämmbarkeitsverbesserer, rückfettende Agenzien und Entschäumer.

Gegenstand der Erfindung ist auch ein Verfahren zur Herstellung von Haarbehandlungsgelen sowie die durch dieses Verfahren hergestellten Gele. Bei dem Herstellungsverfahren wird zunächst eine Gelmasse auf wässriger Lösungsmittelbasis hergestellt wird mit einem Gehalt an
(A) mindestens einem ersten Polymer, ausgewählt aus Hydrokolloiden auf Basis von nichtionischen oder anionischen Polysacchariden;
(B) mindestens einem zweiten Polymer, ausgewählt aus Homo- oder Copolymeren, aufgebaut aus mindestens einer Monomerart, die ausgewählt ist aus Acryl- oder Methacrylamidoalkylsulfonsäuren oder deren Salzen und
(C) mindestens einem dritten, von (A) und (B) verschiedenen Polymer, ausgewählt aus amphiphilen Polymeren.

Anschließend wird die Gelmasse bei Temperaturen oberhalb Raumtemperatur (25°C) gelagert. Durch die Lagerung bei erhöhter Temperatur stellt sich eine neue Konsistenz ein. Die Lagerung erfolgt idealerweise so lange, bis sich die neue Konsistenz vollständig oder nahezu vollständig eingestellt hat und im wesentlichen keine weiteren Veränderungen mehr wahrnehmbar sind. Lagertemperaturen sind z.B. von 30 bis 80 °C, vorzugsweise 40 bis 70°C oder 45 bis 60 °C. Lagerzeiten sind vorzugsweise mindestens 2 Stunden und maximal 3 Tage z.B. von 5 Stunden bis 1,5 Tagen, insbesondere von 7 Stunden bis 12 h. Die Lagerung bei der erhöhten Temperatur erfolgt vorzugsweise solange, bis eine geleeartige Verfestigung eingesetzt hat. Die sich bei Geleebildung einstellende Konsistenz kann als geleeartig, gallerartig oder Wackelpuddingartig, verfestigt und halbfest beschrieben werden. Das Endprodukt ist dann z.B. gallertartig, durschsichtig, durchscheinend bzw. opak mit inhomogener Anmutung, insbesondere einer stückigen Konsistenz mit makroskopisch sichtbaren Agglomeraten. Es ist aber noch streichfähig und ausreichend gut verreibbar.

Mit den erfindungsgemäßen Mitteln können Haare auf prizipiell zwei verschiedenen Arten behandelt werden. Die eine Anwendungsweise ist diejenige eines sogenannten Finish-Gels. Hierzu wird als letzter (oder einziger) Schritt einer Frisurenerstellung das Mittel auf trockenes Haar aufgebracht und ohne Wärmeeinwirkung in das Haar eingearbeitet. Hierbei können das gesamte Haar oder nur einzelne Partien oder Strähnen behandelt werden. Das behandelte Haar erhält Definition, Struktur, Halt, Wet-Look und/oder Glanz. Eine zweite Anwendungsweise ist diejenige eines sogenannten Föhn-Gels. Die Anwendung erfolgt auf feuchtem Haar, z.B. nach einer Haarwäsche oder nach Anfeuchten des Haares mit Wasser. Das Mittel wird auf das feuchte Haar aufgebracht und in das Haar eingearbeitet. Hierbei können das gesamte Haar oder nur einzelne Partien oder Strähnen behandelt werden. Anschließend werden die behandelten Haare unter Wärmeeinwirkung mit einem Haartrockner getrocknet. Auf diese Weise kann eine volumengebende Wirkung erzielt werden. Gegenstand der Erfindung ist daher auch die Verwendung eines der vorstehend beschriebenen Haarbehandlungsmittel als Föhn-Gel zur Volumenerhöhung einer Frisur und/oder als Finish-Gel zum Strukturieren einer Frisur.

Erfindungsgemäße Haarbehandlungsmittel können folgende Vorteile aufweisen:
Attraktive und ungewöhnliche Konsistenz (ähnlich Wackelpudding); gute Verteilbarkeit; sparsam in der Verwendung; anwendbar als 2-in-1 Gel d.h. sowohl als Föhn-Gel als auch als Finish-Gel; gute Definition und Textur der behandelten Haare; gute Volumenwirkung; wenig Belastung; starke Festigung; keine Rückstände; Haare gut modellierbar; nicht zu schnell trocknend; Glanz gebend; gut geeignet zur Behandlung von lockigem Haar, insbesondere bei Anwendung im feuchten Haar und Lufttrocknung; gut geeignet zur Behandlung von kurzem Haar und zur Erstellung von Föhnfrisuren.

Die nachfolgenden Beispiele sollen den Gegenstand der Erfindung näher erläutern.

### Beispiele

Die nachfolgenden Beispielrezepturen sind sowohl im feuchten als auch im trockenen Haar, d.h. als TWO-IN-ONE Gele anwendbar und erhalten nach Lagerung bei ca. 50°C über Nacht (ca. 10 Stunden) erhalten die Gele eine halbfeste aber nicht schnittfeste, weiche, gut verreibbare, Wackelpudding-artige Konsistenz. Falls eine höhere oder niedrigere Viskosität gewünscht wird, kann das durch Variation des Gehaltes an Pemulen^{®} (Acrylates/C10-30 Alkyl Acrylate Crosspolymer) eingestellt werden.

### Beispiel 1: Haargel

| | |
|---|---|
| 0,3 | Xanthan Gum |
| 1,0 | Ammonium Acryloyldimethyltaurate/VP Copolymer |
| 0,1 | Acrylates/C10-30 Alkyl Acrylate Crosspolymer |
| 0,08 | AMP-95 |
| 1,5 | PVP |
| 2 | Polyquaternium-11 |
| 5 | Glycerin |
| 0,3 | Parfüm |
| 0,3 | Eumulgin^{®} L (PPG-1-PEG-9 Lauryl Glykol Ether) |
| 1,0 | Farbstofflösung |
| Ad 100 | Wasser |

### Beispiel 2: Haargel

| | |
|---|---|
| 0,3 | Xanthan Gum |
| 1,0 | Ammonium Acryloyldimethyltaurate/VP Copolymer |
| 0,2 | Acrylates/C10-30 Alkyl Acrylate Crosspolymer |
| 0,16 | AMP-95 |
| 1,5 | PVP |
| 2 | Polyquaternium-11 |
| 5 | Propylenglykol |
| 0,3 | Parfüm |
| 0,3 | Cremophor^{®} CO 410 (PEG-40 Hydrogenated Castor Oil) |
| 0,5 | Farbstofflösung |
| Ad 100 | Wasser |

### Beispiel 3: Haargel

| | |
|---|---|
| 0,3 | Xanthan Gum |
| 1,0 | Ammonium Acryloyldimethyltaurate/VP Copolymer |
| 0,3 | Acrylates/C10-30 Alkyl Acrylate Crosspolymer |
| 0,23 | AMP-95 |
| 1,5 | PVP |
| 2 | Polyquaternium-11 |
| 5 | Glycerin |
| 0,3 | Parfüm |
| 0,3 | Cremophor^{®} CO 410 (PEG-40 Hydrogenated Castor Oil) |
| 0,7 | Farbstofflösung |
| Ad 100 | Wasser |

### Beispiel 4: Haargel

| | |
|---|---|
| 0,2 | Xanthan Gum |
| 1,2 | Ammonium Acryloyldimethyltaurate/VP Copolymer |
| 0,3 | Acrylates/C10-30 Alkyl Acrylate Crosspolymer |
| 0,23 | AMP-95 |
| 2 | PVP |
| 2,5 | VP/VA Copolymer |
| 5 | Glycerin |
| 0,3 | Parfüm |
| 0,3 | Cremophor CO 410 |
| 0,3 | Farbstofflösung |
| Ad 100 | Wasser |

### Beispiel 5: Haargel

| | |
|---|---|
| 0,5 | Xanthan Gum |
| 0,9 | Ammonium Acryloyldimethyltaurate/VP Copolymer |
| 0,2 | Acrylates/C10-30 Alkyl Acrylate Crosspolymer |
| 1,0 | AMP-95 |
| 1,5 | VA/Crotonates Copolymer |
| 6 | Propylenglykol |
| 0,3 | Parfüm |
| 0,3 | Eumulgin^{®} L |
| 1,0 | Farbstofflösung |
| Ad 100 | Wasser |

### Beispiel 6: Haargel

| | |
|---|---|
| 0,3 | Xanthan Gum |
| 3 | Ammonium Acryloyldimethyltaurate/VP Copolymer |
| 0,1 | Acrylates/C10-30 Alkyl Acrylate Crosspolymer |
| 0,08 | AMP-95 |
| 4 | VP/Methacrylamide/Vinylimidazole Copolymer |
| 4 | Paraffinum Perliquidum |
| 0,3 | Parfüm |
| 0,3 | Cremophor CO 410 |
| 0,7 | Farbstofflösung |
| Ad 100 | Wasser |

### Beispiel 7: Haargel

| | |
|---|---|
| 0,5 | Xanthan Gum |
| 2 | Ammonium Acryloyldimethyltaurate/VP Copolymer |
| 0,5 | Acrylates/C10-30 Alkyl Acrylate Crosspolymer |
| 0,4 | AMP-95 |
| 4 | Polyvinylpyrrolidon |
| 4 | Glycerin |
| 0,3 | Parfüm |
| 0,3 | Cremophor CO 410 |
| 0,6 | Farbstofflösung |
| Ad 100 | Wasser |

## Patentansprüche

1. Verwendung eines Haarbehandlungsmittels als Föhn-Gel zur Volumenerhöhung einer Frisur und/oder als Finish-Gel zum Strukturieren einer Frisur, wobei das Haarbehandlungsmittel einen Gehalt an einer Kombination von
(A) mindestens einem ersten Polymer, ausgewählt aus Hydrokolloiden auf Basis von nichtionischen oder anionischen Polysacchariden;
(B) mindestens einem zweiten Polymer, ausgewählt aus Copolymeren, welche aufgebaut sind aus mindestens einer Monomerart ausgewählt aus Monomeren der allgemeinen Formel H₂C=CH-C(=O)-NH-A-SO₂H oder deren Salzen, wobei A für eine divalente C2- bis C6-Kohlenwasserstoffgruppe steht und wobei diese Monomerart, copolymerisiert ist mit mindestens einem nicht ionischen, radikalisch copolymerisierbarem Monomer, und
(C) mindestens einem dritten, von (A) und (B) verschiedenen Polymer, ausgewählt aus amphiphilen Polymeren enthält,
und wobei das Haarbehandlungsmittel in Form eines Gels vorliegt und die Viskosität mindestens 1000 mPa s beträgt, gemessen als dynamische Viskositätsmessung mit einem HAAKE VT-550 Rheometer, Messkörper SV-DIN bei einer Temperatur von 25°C und einer Schergeschwindigkeit von 50 s⁻¹.

2. Verwendung nach Anspruch 1, **dadurch gekennzeichnet, dass** Polymer (A) in einer Menge von 0,05 bis 5 Gew.%, das Polymer (B) in einer Menge von 0,1 bis 10 Gew.% und/oder das Polymer (C) in einer Menge von 0,01 bis 5 Gew.% vorliegt.

3. Verwendung nach einem der vorhergehenden Ansprüche, **dadurch gekennzeichnet, dass** das Polymer (A), ausgewählt ist aus Xanthan, Gellan, Carboxymethylcellulose, Hydroxypropylcellulose, methylcellulose, Hydroxypropyl-methylcellulose, Hydroxyethylcellulose, Agar-Agar, Carrageenan, Alginate, Johannisbrotbaumkernmehl, Guar Gummi, Gummi arabicum, Karaya Gummi, Tragant, Ghatti Gummi, Pectinen und Hydroxypropylguar.

4. Verwendung nach einem der vorhergehenden Ansprüche, **dadurch gekennzeichnet, dass** das Polymer (A) Xanthan ist.

5. Verwendung nach Anspruch 4, **dadurch gekennzeichnet, dass** das Polymer (B) ein Ammonium Acryloyldimethyltaurat/Vinylpyrrolidon Copolymer ist.

6. Verwendung nach einem der vorhergehenden Ansprüche, **dadurch gekennzeichnet, dass** das Polymer (C) ausgewählt ist aus
(1) Copolymeren von Acryl- oder Methacrylsäure und Monomeren, die mindestens eine Fettkette aufweisen;
(2) Polysacchariden, die mit Gruppen modifiziert sind, die mindestens eine Fettkette aufweisen;
(3) Copolymeren von Maleinsäureanhydrid oder einem ihrer Derivate und Monomeren, die mindestens eine Fettkette aufweisen;
(4) Polyurethanen und deren Derivaten, die Gruppen tragen, die mindestens eine Fettkette aufweisen;
(5) Copolymeren von Crotonsäure und Monomeren, die mindestens eine Fettkette aufweisen;
(6) Copolymeren von N-Vinylpyrrolidon und Monomeren, die mindestens eine Fettkette aufweisen
(7) nichtionischen Copolymeren von acryl- oder Methacrylsäurealkylestern mit G1-6-Alkylgruppen und amphiphilen Monomeren mit Fettkette;
(8) nichtionischen Copolymeren von hydrophilen Acrylaten oder Methacrylaten und hydrophoben Monomeren mit Fettkette.

7. Verwendung nach Anspruch 6, **dadurch gekennzeichnet, dass** das Polymer (C) ausgewählt ist aus vernetzten oder unvernetzten Copolymeren, hergestellt aus mindestens einer ersten Monomerart, welche ausgewählt ist aus Acrylsäure und Methacrylsäure und mindestens einer zweiten Monomerart, welche ausgewählt ist aus C8- bis C30-Alkylestern der Acrylsäure und C8- bis C30-Alkylestern der Methacrylsäure.

8. Verwendung nach einem der vorhergehenden Ansprüche, **dadurch gekennzeichnet, dass** das Haarbehandlungsmittel einen Gehalt aufweist an
(A) 0,1 bis 1 Gew.% Xanthan,
(B) 0,2 bis 5 Gew.% Ammonium Acryloyldimethyltaurat/Vinylpyrrolidon Copolymer und
(C) 0,05 bis 2 Gew.% an vernetzten Copolymeren, hergestellt aus mindestens einer ersten Monomerart, welche ausgewählt ist aus Acrylsäure und Methacrylsäure und mindestens einer zweiten Monomerart, welche ausgewählt ist aus C10- bis C30-Alkylestern der Acrylsäure und C10- bis C30-Alkylestern der Methacrylsaure.

9. Verwendung nach einem der vorhergehenden Ansprüche, **dadurch gekennzeichnet, dass** das Haarbehandlungsmittel zusätzlich mindestens ein filmbildendes oder haarfestigendes Polymer enthält.

10. Verwendung nach Anspruch 9, **dadurch gekennzeichnet, dass** das zusätzliche haarfestigende Polymer ausgewählt ist aus Terpolymeren aus Acrylsäure, Ethylacrylat und N-tert-Butylacrylamid; vernetzten oder unvernetzten Vinylacetat/Crotonsäure Copolymeren; Terpolymeren aus tert.-Butylacrylat, Ethylacrylat und Methacrylsäure; Natriumpolystyrolsulfonat; Copolymeren aus Vinylacetat, Crotonsäure und Vinylpropionat; Copolymeren aus Vinylacetat, Crotonsäure und Vinylneodecanoat; Aminomethylpropanol-Acrylat Copolymeren; Copolymeren aus vinylpyrrolidon und mindestens einem weiteren Monomer ausgewählt aus Acrylsäure, Methacrylsäure, Acrylsäureestern und Methacrylsäureestern; Copolymeren aus Methylvinylether und Maleinsäuremonoalkylestein; Aminomethylpropanolsalze von Copolymeren aus Allylmethacrylat und mindestens einem weiteren Monomer ausgewählt aus Acrylsäure, Methacrylsäure, Acrylsäureestern und Methacrylsäureestern;Copolymeren aus Vinylacetat, Mono-n-butylmaleat und Isobornylacrylat; Copolymeren aus zwei oder mehr Monomeren ausgewählt aus Acrylsäure, Methacrylsäure, Acrylsäureestern und Methacrylsäureestern, Copolymeren aus Octylacrylamid und mindestens einem Monomeren ausgewählt aus Acrylsäure, Methacrylsäure, Acrylsäureestern und Methacrylsäureestern; Polyestern aus Diglycol, Cyclohexandimethanol, Isophtalsäure und Sulfoisophtalsäure; Copolymer aus Vinylcaprolactam, Vinylpyrrolidon und Dimethylaminoethylmethacrylat; Copolymeren aus Vinylpyrrolidon,und Dimethylaminoethylinethacrylat, Copolymeren aus Vinylpyrrolidon, Vinylcaprolactam und Dimethylaminopropylacrylamid; Copolymeren aus Octylacrylamid, Acrylsäure, Butylaminoethylmethacrylat; Methylmethacrylat und Hydroxypropylmethacrylat; Copolymeren aus Laurylacrylat, Stearylacrylat ; Polyvinylpyrrolidon, Polyvinylcaprolactam, Vinylpyrrolidon/Vinylacetat CopolymerenPoly-Vinylalkohol, Isobutylen/Ethylmaleimid/Hydroxyethylmaleimid Copolymer; Copolymyeren aus Vinylpyrrolidon, Vinylacetat und Vinylpropionat; copolymer aus Vinylpyrrolidon, Methacrylamid und Vinylimidazol;hydrolysierte Maisstärke.

11. Verwendung nach einem der vorhergehenden Ansprüche, **dadurch gekennzeichnet, dass** das Haarbehandlungsmittel zusätzlich mindestens eine Fettkomponente enthält.

12. Verwendung nach Anspruch 11, **dadurch gekennzeichnet, dass** die zusätzliche Fettkomponente ausgewählt ist aus
- Silikonverbindungen, welche ausgewählt sind aus Silikonwachsen, Silikonölen, cyclischen Dimethylsiloxanen, linearen Polydimethylsiloxanen, Blockpolymeren aus Polydimethylsiloxan und Polyethylenoxid und/oder Polypropylenoxid, Polydimethylsiloxanen mit end- oder seitenständigen Polyethylenoxid- oder Polypropylenoxidresten, Polydimethylsiloxanen mit endständigen Hydroxylgruppen,phenylsubstituierten Polydimethylsiloxanen, Silikonemulsionen, Silkonelastomeren, Silikongums und aminosubstituierten Silikonen und
- Ölen und Wachsen, welche ausgewählt sind aus Paraffin-Wachsen, Polyolefinwachsen; Wollwachs, Wollwachsalkoholen, Gandelillawachs, Olivenwachs, Carnaubawachs, Japanwachs, Apfelwachs, gehärteten Fetten, Fettsäureestern, Fettsäureglyceriden, Fettsäuretriglyceriden, Polyethylenglykolwachsen,/Mineralölen, Isoparaffinölen, Paraffinölen, Squalan, Sonnenblumenöl, Kokosöl Rizinusöl, Lanolinöl, Jojobaöl, Maisöl und Sojaöl

13. Verfahren zur Herstellung eines Haarbehandlungsgels gemäß Anspruch 1, wobei zunächst eine Gelmasse auf wässriger Lösungsmittelbasis hergestellt wird mit einem Gehalt an
(A) mindestens einem ersten Polymer, ausgewählt aus Hydrokolloiden auf Basis von nichtionischen oder anionischen Polysacchariden;
(B) mindestens einem zweiten Polymer, ausgewählt aus Copolymeren, welche aufgebaut sind aus mindestens einer Monomerart ausgewählt aus Monomeren der allgemeinen Formel H₂C=CH-C(=O)-NH-A-SO₃ oder deren Salzen wobei A für eine divalente C2- bis C6-Kohlenwässerstoffgruppe steht und wobei diese Monomerart copolymerisiert ist mit mindestens einem nicht ionischen, radikalisch copolymerisierbarem Monomer, und
(C) mindestens einem dritten, von (A) und (B) verschiedenen Polymer, ausgewählt aus amphiphilen Polymeren
und anschließend die Gelmasse bei Temperaturen oberhalb Raumtemperatur (25°C) gelagert wird bis eine geleeartige Verfestigung eintritt.

## Claims

1. Use of a hair treatment composition as blow-drying gel for increasing the volume of a hairstyle and/or as finish gel for the structuring of a hairstyle, where the hair treatment composition comprises a content of a combination of
(A) at least one first polymer selected from hydrocolloids based on nonionic or anionic polysaccharides,
(B) at least one second polymer selected from copolymers which are composed of at least one monomer type selected from monomers of the general formula H₂C=CH-C(=O)-NH-A-SO₃H or salts thereof, where A is a divalent C2- to C6-hydrocarbon group and where this monomer type is copolymerized with at least one nonionic, free-radically copolymerizable monomer, and
(C) at least one third polymer, different from (A) and (B), selected from amphiphilic polymers, and where the hair treatment composition is in the form of a gel and the viscosity is at least 1000 mPa s, measured as dynamic viscosity measurement using a HAAKE VT-550 rheometer, measurement body SV-DIN at a temperature of 25°C and a shear rate of 50 s⁻¹.

2. Use according to Claim 1, **characterized in that** polymer (A) is present in an amount of from 0.05 to 5% by weight, the polymer (B) is present in an amount of from 0.1 to 10% by weight and/or the polymer (C) is present in an amount of from 0.01 to 5% by weight.

3. Use according to one of the preceding claims, **characterized in that** the polymer (A) is selected from xanthan, gellan, carboxymethylcellulose, hydroxypropylcellulose, methylcellulose, hydroxypropylmethylcellulose, hydroxyethylcellulose, agar agar, carrageenan, alginates, carob seed flour, guar gum, gum arabic, karaya gum, tragacanth, ghatti gum, pectins and hydroxypropylguar.

4. Use according to one of the preceding claims, **characterized in that** the polymer (A) is xanthan.

5. Use according to Claim 4, **characterized in that** the polymer (B) is an ammonium acryloyldimethyltaurate/vinylpyrrolidone copolymer.

6. Use according to one of the preceding claims, **characterized in that** the polymer (C) is selected from
(1) copolymers of acrylic acid or methacrylic acid and monomers which have at least one fatty chain;
(2) polysaccharides which are modified with groups which have at least one fatty chain;
(3) copolymers of maleic anhydride or one of its derivatives and monomers which have at least one fatty chain;
(4) polyurethanes and derivatives thereof which carry groups which have at least one fatty chain;
(5) copolymers of crotonic acid and monomers which have at least one fatty chain;
(6) copolymers of N-vinylpyrrolidone and monomers which have at least one fatty chain;
(7) nonionic copolymers of acrylic acid or methacrylic acid alkyl esters having C1-6-alkyl groups and amphiphilic monomers with fatty chain;
(8) nonionic copolymers of hydrophilic acrylates or methacrylates and hydrophobic monomers with fatty chain.

7. Use according to Claim 6, **characterized in that** the polymer (C) is selected from crosslinked or uncrosslinked copolymers prepared from at least one first monomer type which is selected from acrylic acid and methacrylic acid and at least one second monomer type which is selected from C8- to C30-alkyl esters of acrylic acid and C8- to C30-alkyl esters of methacrylic acid.

8. Use according to one of the preceding claims, **characterized in that** the hair treatment composition has a content of
(A) 0.1 to 1% by weight of xanthan,
(B) 0.2 to 5% by weight of ammonium acryloyldimethyltaurate/vinylpyrrolidone copolymer and
(C) 0.05 to 2% by weight of crosslinked copolymers prepared from at least one first monomer type which is selected from acrylic acid and methacrylic acid and at least one second monomer type which is selected from C10- to C30-alkyl esters of acrylic acid and C10- to C30-alkyl esters of methacrylic acid.

9. Use according to one of the preceding claims, **characterized in that** the hair treatment composition additionally comprises at least one film-forming or hair-setting polymer.

10. Use according to Claim 9, **characterized in that** the additional hair-setting polymer is selected from terpolymers of acrylic acid, ethyl acrylate and N-tert-butylacrylamide; crosslinked or uncrosslinked vinyl acetate/crotonic acid copolymers; terpolymers of tert-butyl acrylate, ethyl acrylate and methacrylic acid; sodium polystyrenesulphonate; copolymers of vinyl acetate, crotonic acid and vinyl propionate; copolymers of vinyl acetate, crotonic acid and vinyl neodecanoate; aminomethylpropanol acrylate copolymers; copolymers of vinylpyrrolidone and at least one further monomer selected from acrylic acid, methacrylic acid, acrylic acid esters and methacrylic acid esters; copolymers of methyl vinyl ether and maleic acid monoalkyl esters; aminomethylpropanol salts of copolymers of allyl methacrylate and at least one further monomer selected from acrylic acid, methacrylic acid, acrylic acid esters and methacrylic acid esters; copolymers of vinyl acetate, mono-n-butyl maleate and isobornyl acrylate; copolymers of two or more monomers selected from acrylic acid, methacrylic acid, acrylic acid esters and methacrylic acid esters, copolymers of octylacrylamide and at least one monomer selected from acrylic acid, methacrylic acid, acrylic acid esters and methacrylic acid esters; polyesters of diglycol, cyclohexanedimethanol, isophthalic acid and sulphoisophthalic acid; copolymers of vinylcaprolactam, vinylpyrrolidone and dimethylaminoethyl methacrylate; copolymers of vinylpyrrolidone and dimethylaminoethyl methacrylate, copolymers of vinylpyrrolidone, vinylcaprolactam and dimethylaminopropylacrylamide; copolymers of octylacrylamide, acrylic acid, butylaminoethyl methacrylate, methyl methacrylate and hydroxypropyl methacrylate; copolymers of lauryl acrylate, stearyl acrylate, polyvinylpyrrolidone, polyvinylcaprolactam, vinylpyrrolidone/vinyl acetate copolymers, polyvinyl alcohols, isobutylene/ethylmaleimide/hydroxyethylmaleimide copolymer; copolymers of vinylpyrrolidone, vinyl acetate and vinyl propionate; copolymer of vinylpyrrolidone, methacrylamide and vinylimidazole; hydrolysed corn starch.

11. Use according to one of the preceding claims, **characterized in that** the hair treatment composition additionally comprises at least one fatty component.

12. Use according to Claim 11, **characterized in that** the additional fatty component is selected from
- silicone compounds which are selected from silicone waxes, silicone oils, cyclic dimethylsiloxanes, linear polydimethylsiloxanes, block polymers of polydimethylsiloxane and polyethylene oxide and/or polypropylene oxide, polydimethylsiloxanes with terminal or lateral polyethylene oxide or polypropylene oxide radicals, polydimethylsiloxanes with terminal hydroxyl groups, phenyl-substituted polydimethylsiloxanes, silicone emulsions, silicone elastomers, silicone gum and amino-substituted silicones and oils and waxes which are selected from paraffin waxes, polyolefin waxes, wool wax, wool wax alcohols, candelilla wax, olive wax, carnauba wax, Japan wax, apple wax, hardened fats, fatty acid esters, fatty acid glycerides, fatty acid triglycerides, polyethylene glycol waxes, mineral oils, isoparaffin oils, paraffin oils, squalane, sunflower oil, coconut oil, castor oil, lanolin oil, jojoba oil, corn oil and soya oil.

13. Process for the preparation of a hair treatment gel according to Claim 1, where firstly a gel mass based on an aqueous solvent is prepared with a content of
(A) at least one first polymer selected from hydrocolloids based on nonionic or anionic polysaccharides,
(B) at least one second polymer selected from copolymers which are composed of at least one monomer type selected from monomers of the general formula H₂C=CH-C(=O)-NH-A-SO₃H or salts thereof, where A is a divalent C2- to C6-hydrocarbon group and where this monomer type is copolymerized with at least one nonionic, free-radically copolymerizable monomer, and
(C) at least one third polymer, different from (A) and (B), selected from amphiphilic polymers,
and then the gel mass is stored at temperatures above room temperature (25°C) until a jelly-like solidification occurs.

## Revendications

1. Utilisation d'une composition de traitement capillaire sous forme de gel pour séchage au sèche-cheveux, pour l'augmentation de volume d'une coiffure et/ou sous forme de gel de finition pour la structuration d'une coiffure, la composition de traitement capillaire ayant une teneur en une association de
(A) au moins un premier polymère choisi parmi des hydrocolloïdes à base de polysaccharides anioniques ou non ioniques ;
(B) au moins un deuxième polymère choisi parmi les polymères qui sont constitués d'au moins un type de monomère choisi parmi les monomères de formule générale H₂C=CH-C(=O)-NH-A-SO₃H ou leurs sels, A représentant un groupe hydrocarboné divalent en C₂ à C₆, et ce type de monomère étant copolymérisé avec au moins un monomère non ionique, susceptible de polymérisation radicalaire, et
(C) au moins un troisième polymère, différent de (A) et (B), choisi parmi des polymères amphiphiles,
et la composition de traitement capillaire se trouvant sous forme d'un gel et la viscosité étant d'au moins 1 000 mPa.s, mesurée en tant que mesure dynamique de la viscosité au moyen d'un rhéomètre HAAKE VT-550, élément de mesure SV-DIN, à une température de 25 °C et à une vitesse de cisaillement de 50 s⁻¹.

2. Utilisation selon la revendication 1, **caractérisée en ce que** le polymère (A) est présent en une quantité de 0,05 à 5 % en poids, le polymère (B) est présent en une quantité de 0,1 à 10 % en poids et/ou le polymère (C) est présent en une quantité de 0,01 à 5 % en poids.

3. Utilisation selon l'une quelconque des revendications précédentes, **caractérisée en ce que** le polymère (A) est choisi parmi le xanthane, le gellane, la carboxyméthylcellulose, l'hydroxypropylcellulose, la méthylcellulose, l'hydroxypropyl-méthylcellulose, l'hydroxyéthylcellulose, l'agar-agar, le carraghénane, les alginates, la farine de graines de caroube, la gomme guar, la gomme arabique, la gomme karaya, la gomme adragante, la gomme ghatti, les pectines et l'hydroxypropylguar.

4. Utilisation selon l'une quelconque des revendications précédentes, **caractérisée en ce que** le polymère (A) est le xanthane.

5. Utilisation selon la revendication 4, **caractérisée en ce que** le polymère (B) est un copolymère acryloyldiméthyltaurate d'ammonium/vinylpyrrolidone.

6. Utilisation selon l'une quelconque des revendications précédentes, **caractérisée en ce que** le polymère (C) est choisi parmi
(1) des copolymères d'acide acrylique ou méthacrylique et de monomères qui comportent au moins une chaîne grasse ;
(2) des polysaccharides qui sont modifiés par des groupes qui comportent au moins une chaîne grasse;
(3) des copolymères d'anhydride maléique ou d'un de ses dérivés et de monomères qui comportent au moins une chaîne grasse;
(4) des polyuréthannes et leurs dérivés qui portent des groupes qui comportent au moins une chaîne grasse ;
(5) des copolymères d'acide crotonique et de monomères qui comportent au moins une chaîne grasse;
(6) des copolymères de N-vinylpyrrolidone et de monomères qui comportent au moins une chaîne grasse ;
(7) des copolymères non ioniques d'esters alkyliques d'acide acrylique ou méthacrylique, comportant des groupes alkyle en C₁-C₆, et de monomères amphiphiles à chaîne grasse ;
(8) des copolymères non ioniques d'acrylates ou méthacrylates hydrophiles et de monomères hydrophobes à chaîne grasse.

7. Utilisation selon la revendication 6, **caractérisée en ce que** le polymère (C) est choisi parmi des copolymères réticulés ou non réticulés, préparés à partir d'au moins un premier type de monomère qui est choisi parmi l'acide acrylique et l'acide méthacrylique et d'au moins un deuxième type de monomère qui est choisi parmi des esters alkyliques en C₈-C₃₀ de l'acide acrylique et des esters alkyliques en C₈-C₃₀ de l'acide méthacrylique.

8. Utilisation selon l'une quelconque des revendications précédentes, **caractérisée en ce que** la composition de traitement capillaire a une teneur
(A) de 0,1 à 1 % en poids en xanthane,
(b) de 0,2 à 5 % en poids en copolymère acryloyldiméthyltaurate d'ammonium/vinylpyrrolidone et
(c) de 0,05 à 2 % en poids en copolymères réticulés, préparés à partir d'au moins un premier type de monomère qui est choisi parmi l'acide acrylique et l'acide méthacrylique et d'au moins un deuxième type de monomère qui est choisi parmi des esters alkyliques en C₁₀-C₃₀ de l'acide acrylique et des esters alkyliques en C₁₀-C₃₀ de l'acide méthacrylique.

9. Utilisation selon l'une quelconque des revendications précédentes, **caractérisée en ce que** la composition de traitement capillaire en outre contient au moins un polymère filmogène ou fixant les cheveux.

10. Utilisation selon la revendication 9, **caractérisée en ce que** le polymère supplémentaire fixant les cheveux est choisi parmi les terpolymères d'acide acrylique, acrylate d'éthyle et N-tert-butylacrylamide ; les copolymères acétate de vinyle/acide crotonique réticulés ou non réticulés ; les terpolymères d'acrylate de tert-butyle, acrylate d'éthyle et acide méthacrylique ; le polystyrènesulfonate de sodium ; les copolymères d'acétate de vinyle, acide crotonique et propionate de vinyle ; les copolymères d'acétate de vinyle, acide crotonique et néodécanoate de vinyle ; les copolymères aminométhylpropanol/acrylate ; les copolymères de vinylpyrrolidone et d'au moins un autre monomère choisi parmi l'acide acrylique, l'acide méthacrylique, des esters d'acide acrylique et des esters d'acide méthacrylique ; les copolymères d'oxyde de méthyle et de vinyle et de maléates de monoalkyle ; les sels avec l'aminométhylpropanol de copolymères de méthacrylate d'allyle et d'au moins un autre monomère choisi parmi l'acide acrylique, l'acide méthacrylique, des esters d'acide acrylique et des esters d'acide méthacrylique ; les copolymères d'acétate de vinyle, maléate de mono-n-butyle et acrylate d'isobornyle ; les copolymères de deux ou plus de deux monomères choisis parmi l'acide acrylique, l'acide méthacrylique, des esters d'acide acrylique et des esters d'acide méthacrylique ; les copolymères d'octylacrylamide et d'au moins un monomère choisi parmi l'acide acrylique, l'acide méthacrylique, des esters d'acide acrylique et des esters d'acide méthacrylique ; les polyesters de diglycol, cyclohexanediméthanol, acide isophtalique et acide sulfo-isophtalique ; un copolymère de vinylcaprolactame, vinylpyrrolidone et méthacrylate de diméthylaminoéthyle, les copolymères de vinylpyrrolidone et méthacrylate de diméthylaminoéthyle, les copolymères de vinylpyrrolidone, vinylcaprolactame et diméthylaminopropylacrylamide, les copolymères d'octylacrylamide, acide acrylique, méthacrylate de butylaminoéthyle, méthacrylate de méthyle et méthacrylate d'hydroxypropyle ; les copolymères d'acrylate de lauryle, acrylate de stéaryle, polyvinylpyrrolidone, polyvinylcaprolactame, les copolymères vinylpyrrolidone/acétate de vinyle, le poly(alcool vinylique), un copolymère isobutylène/éthylmaléimide/hydroxyéthylmaléimide ; les copolymères de vinylpyrrolidone, acétate de vinyle et propionate de vinyle ; un copolymère de vinylpyrrolidone, méthacrylamide et vinylimidazole ; l'amidon de maïs hydrolysé.

11. Utilisation selon l'une quelconque des revendications précédentes, **caractérisée en ce que** la composition de traitement capillaire en outre contient au moins un composant gras.

12. Utilisation selon la revendication 11, **caractérisée en ce que** le composant gras supplémentaire est choisi parmi
- des composés silicone qui sont choisis parmi des cires de silicone, des huiles de silicone, des diméthylsiloxanes cycliques, des polydiméthylsiloxanes linéaires, des polymères séquencés de polydiméthylsiloxane et polyoxyéthylène et/ou polyoxypropylène, des polydiméthylsiloxanes comportant des radicaux polyoxyéthylène ou polyoxypropylène latéraux ou en bout de chaîne, des polydiméthylsiloxanes à groupes hydroxy en bout de chaîne, des polydiméthylsiloxanes substitués par phényle, des émulsions de silicone, des élastomères de silicone, des gommes de silicone et des silicones substitués par amino et
- des huiles et cires qui sont choisies parmi des cires de paraffine, des cires polyoléfiniques, la lanoline, les alcolanums, la cire de candelilla, la cire d'olive, la cire de carnauba, la cire du Japon, la cire de pomme, des graisses durcies, des esters d'acides gras, des glycérides d'acides gras, des triglycérides d'acides gras, des cires de polyéthylèneglycol, des huiles minérales, des huiles isoparaffiniques, des huiles de paraffine, le squalane, l'huile de tournesol, l'huile de coprah, l'huile de ricin, l'huile de lanoline, l'huile de jojoba, l'huile de maïs et l'huile de soja.

13. Procédé pour la préparation d'un gel de traitement capillaire selon la revendication 1, dans lequel d'abord on prépare une masse de gel à base de solvant aqueux, ayant une teneur en
(A) au moins un premier polymère choisi parmi des hydrocolloïdes à base de polysaccharides anioniques ou non ioniques;
(B) au moins un deuxième polymère choisi parmi les polymères qui sont constitués d'au moins un type de monomère choisi parmi les monomères de formule générale H₂C=CH-C(=O)-NH-A-SO₃H ou leurs sels, A représentant un groupe hydrocarboné divalent en C₂ à C₆, et ce type de monomère étant copolymérisé avec au moins un monomère non ionique, susceptible de polymérisation radicalaire, et
(C) au moins un troisième polymère, différent de (A) et (B), choisi parmi des polymères amphiphiles,
et ensuite on porte la masse de gel à des températures supérieures à la température ambiante (25°C), jusqu'à ce qu'apparaisse une solidification de type gelée.
